# EUROPEAN PATENT APPLICATION

(11) **EP 1 127 583 A2**
(43) Date of publication of application: **29.08.2001**
(21) Application number: 00127113.9
(22) Date of filing: 11.12.2000
(51) Int. Cl.: A61M 16/08

(54) **Conduit with transmission line**

(30) Priority: 24.02.2000 SE 0000605
(71) Applicant: Siemens-Elema AB, 171 95 Solna 1 (SE)
(72) Inventor: von Bahr, Pontus, 12237 Enskede (SE)

(57) **Abstract**

A conduit (2,22) connectable in a fluid transfer system to provide for the through flow of a fluid moving between a transfer device (40) and internal a patient includes a transmission line (14,36) incorporated therewith having a distal end for signal communication with a signal generator (28) and a proximal end connected to a terminal (18a,b) on a mating surface (20) of a female connector (8) and arranged thereon to establish a signal transmission path with a terminal (48a,b) on a mating surface (46) of a second conduit, such as a port (44) of the fluid transfer device (40), as the mating surfaces (20;46) engage to establish fluid communication between the conduit (2,22) and the port (44).

## Description

The present invention relates to a conduit used in a fluid flow path connecting a fluid transfer device to a patient and to a fluid transfer system incorporating the same.

Very often a fluid transfer device, such as a liquid ventilator which transfers a breathing liquid to and from a patient; a gas ventilator or respirator which transfers gas to and from a patient; or a dialysis machine which transfers blood to and from a patient, which is used in patient care needs to communicate with external elements such as sensors or valves connected to a fluid flow path linking the device to internal a patient.

Such communication may be necessary to enable the device to control an external valve in order to regulate the fluid flow within the flow path. Alternatively or additionally such communication may be necessary to provide the device with information from sensors in communication with the fluid which is useable internal the device for control purposes or which is useable in a display system to provide a sensible indication of the efficacy of the care being provided.

This requires that in addition to the fluid conduits which comprise the fluid flow path one or more transmission lines, such as electrical wires or optical cables, must be provided along which signals may be passed. These lines often induce additional installation work for the user, more difficult transportation of the patient, and an increased risk of accidental user or patient interaction with the lines which might cause involuntary disconnection of the external elements or a potential heath hazard.

It is known to provide a fluid conduit having incorporated therein an fibre optical transmission line having a proximal end for signal communication with a tv camera and a distal end for receiving light reflected from a region to be imaged using the camera.

It is an aim of the present invention to alleviate at least one of the problems associated with the presence of transmission lines.

According to a first aspect of the present invention there is provided a conduit as described in and characterised by the present claim 1. By providing a transmission line, for example an optical or an electrical transmission line, which is incorporated with the fluid conduit and which is terminated at one or both the proximal and the distal ends of the conduit by an interface adapted to establish a concomitant signal and fluid coupling of the conduit with another conduit within a fluid transfer system then the potential for accidental user or patient interaction with the line is reduced as is the installation work for a user and the possibility of failing to establish a signal connection.

Usefully, the line is incorporated so as to substantially maintain the flexibility of the conduit, such as for example by being helically wound to follow the contours of a 'concertina' hose often used as a flexible conduit within a gas flow path of a ventilator system.

According to a second aspect of the present invention there is provided a fluid transfer system as described in and characterised by the present claim 4. The inclusion in the system of a conduit according the first aspect of the present invention provides the advantages to the system associated with the conduit, as disclosed above.

Usefully, the transfer device includes a port through which fluid may be transferred between internal and external the device having at an externally accessible surface a terminal connected to the processing unit and an interface is provided for establishing concomitantly a coupling of the transmission line with the terminal of the port and a fluid coupling of the proximal end of the conduit with the port. In this manner both signal and fluid communication may be established between the conduit and the device in a single operation.

Advantageously, a detector may be provided connected to the input terminal of the transfer device and adapted to detect the connection of the sensor to the transmission line. This allows an automatic confirmation of a correct coupling to be made or an automatic re-configuration of the system to accept signals from a sensor connected using conventional transmission lines.

These and other advantages will be elucidated by consideration of the following description of exemplary embodiments of the present invention, made with reference to the drawings of the accompanying figures, of which:

Fig. 1 shows a schematic representation of a conduit according to the present invention.

Fig. 2 shows a schematic representation of a further embodiment of a conduit according to the present invention

Fig. 3 shows elements of a fluid transfer system according to the present invention incorporating the conduits of Figs. 1 and 2.

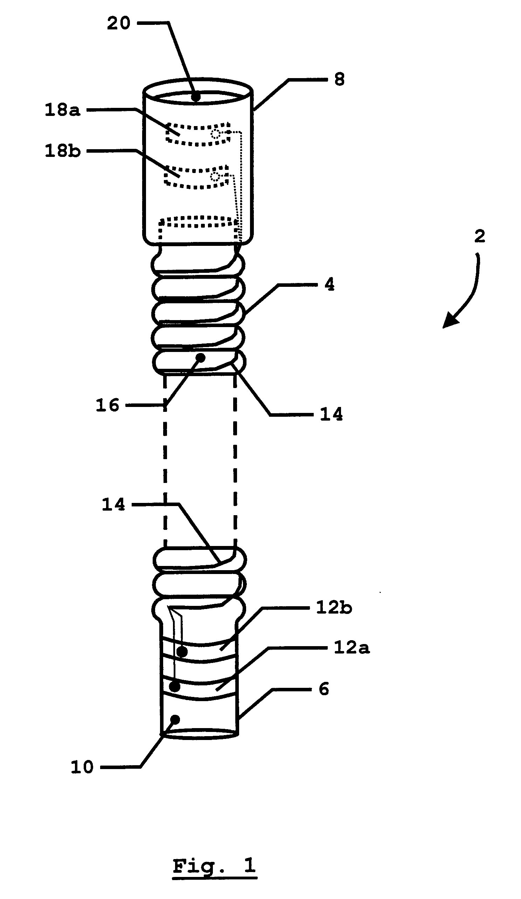

Considering now Fig. 1, a conduit 2 according to the present invention comprises a length of flexible gas tubing 4 having, at opposite ends, a male interface 6 and a female interface 8 for interconnecting with other gas conduits.

The male interface 6 may, for example, be formed of a rigid plastics material over which a female sealing piece of another conduit may be fit and has two electrical contacts 12a,b on an external mating surface 10. These contacts 12a,b are connected to respective electrical wires of a two-core cable 14. The cable 14 is helically wound about an external surface 16 of the gas tubing 4 and opposite ends of the wires are connected to respective electrical contacts 18a,b which are located on an internal mating surface 20 of the female interface 8 which may be formed of a flexible rubber material to form a gas seal when push-fit with a co-operating male member of another conduit. In this manner an electrical signal transmission path is provided along the length of the tubing 4, between corresponding pairs of externally accessible contacts 12a,18a and 12b,18b.

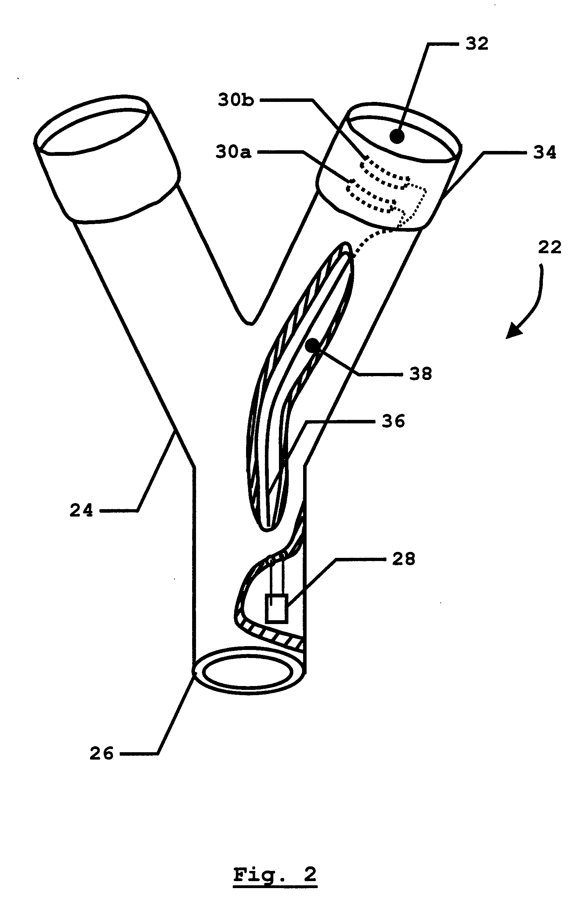

Considering now Fig. 2, a conduit 22 according to the present invention comprises a rigid Y-piece connector 24 having located within the common stem-section 26 a sensor 28, such as a solid state pressure transducer or a flow meter. Electrical contacts 30a,b are located on an internal mating surface 32 of an interface 34 which is here shown in the form of a female connector adapted to form a gas seal when mated with a co-operating male member of another conduit. A cable 36 is provided to establish an electrical signal transmission path between the sensor 28 and the contacts 30a,b and is incorporated with the conduit 22 within a channel 38 in a side wall of the Y-piece connector 24.

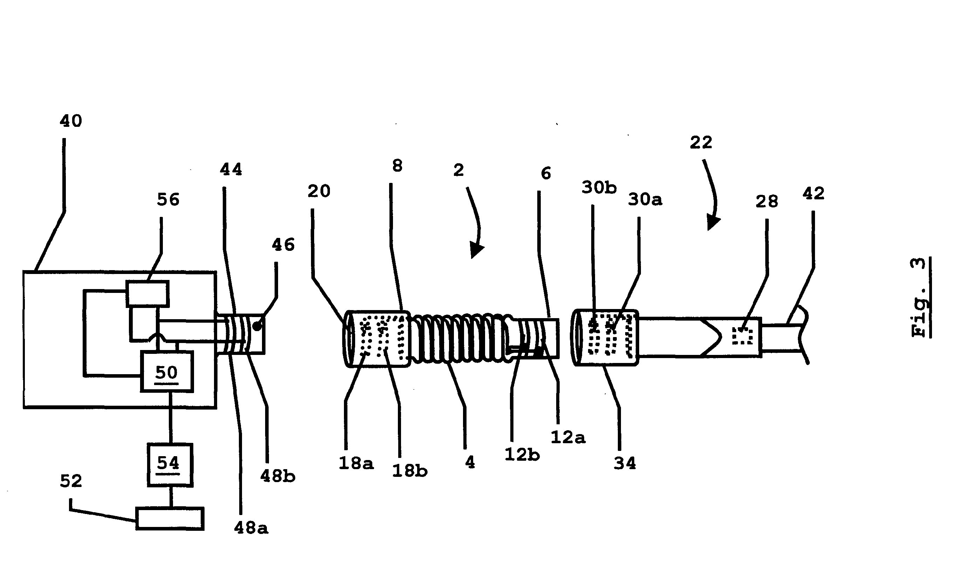

An example of a fluid transfer system according to the present invention for the transfer of gas between the airways of a patient (not shown) and a fluid transfer device (ventilator 40) of the system is shown in Fig. 3. Conduits 2,22 are provided interconnectable with an endotracheal tube 42 (shown in part) to form a flow path for gas passing between the ventilator 40 and a patient's airways (not shown) into which the endotracheal tube 42 will be inserted in use.

The ventilator 40 includes a gas port 44 which has on an external mating surface 46 two electrical contacts 48a,b intended for connection with the contacts 18a,b provided on the inside of the female interface 8 at a proximal end of the flexible tubing 4. The contacts 48a,b and 18a,b are disposed on their respective surfaces 46,20 such that when the female interface 8 is push fit over the port 44 to thereby form a gas tight connection the contacts 48a,b and 18a,b will concomitantly establish an electrical connection. Preferably one or both sets of contacts 48a,b and 18a,b are provided as rings 48a,b about the periphery of the corresponding mating surface 46. This enables signal communication to be established between the sets of contacts 48a,b and 18a,b irrespective of the rotational orientation of the conduit 2.

Similarly, when the male interface 6 at the distal end of the tubing 4 is interconnected with the female interface 34 to establish a gas tight connection then an electrical connection is automatically established between the electrical contacts 12a,b and 30a,b (either or both of which may again be formed as ring contacts 12a,b) of their respective interfaces 6,34. Thus a signal path between a sensor 28, here within the Y-piece conduit 22, and a processing unit 50, here within the ventilator 40 and connected to the contacts 48a,b of the port 44, is automatically established as the flow path for connecting the ventilator 40 to a patient's airways is established.

The processing unit 50 functions to control the operation of the ventilator 40 to regulate gas transfer so as to provide a desired breathing pattern, as known in the art of mechanical ventilation, for example to maintain flow parameters as set by a user via an interface 52. Also as common in the art, the processing unit may process the output from the sensor 28 to provide a visible indication on a display 54 of monitored properties of the gas which may be used to determine the efficacy of the ventilation therapy being provided by the ventilator 40.

A detector 56 may be provided connected to the contacts 48a,b in order to detect whether or not a sensor is connected which may also indicate whether or not the gas/electrical interconnections between the port 44 and the interface 8 at the proximal end of the tubing 4 and between the interface 6 at the distal end of the tubing 4 and the interface 34 of the Y-piece conduit 22 have been properly established. This can be done by adapting the detector 56 to monitor the resistance of the transmission path essentially between the sensor 28 and the contact 48a,b of the port 44.

The absence of a sensor 28 or of proper interconnections can be reported to the user via the display 54, as an output either directly from the detector 56 or from the processing unit 50 to which the detector 56 may also be connected. The processing unit 50 may then be configured to inhibit the operation of the ventilator 40 or to use conventional sensor inputs in response to the report from the detector 56.

It will be appreciated by those skilled in the art that the embodiments of the present invention provided above may be modified in a number of non-inventive ways whilst still remaining within the invention as claimed. For example the signal transmission lines may be used to provide bidirectional communication or for communication with devices other than sensors, such as flow control valves. Additionally, more than one transmission line may be incorporated with the conduit and the one or more transmission lines may be optical fibre or acoustic waveguide.

## Claims

1. A conduit (2;22) connectable in a fluid transfer system to provide for the through flow of a fluid moving between a transfer device (40) and internal a patient having incorporated therewith a transmission line (14;36) having a proximal end for signal communication with one or other of a signal generator (28) and a signal receiver (50) and a distal end for signal communication with a complementary one or other of the signal receiver (50) and the signal generator (28) to provide a signal transmission path therebetween
**characterised in that** an interface (6;8;34) is provided in permanent connection with an end of the conduit (2;22) and adapted to establish concomitantly a coupling of the transmission line (14;36) with a signal terminal (48a,b; 12a,b)) of a second conduit (44,2) and a fluid coupling of the conduit (2;22) with the second conduit (44,2).

2. A conduit (2) as claimed in claim 1
**characterised in that** the interface (8) comprises a male or female (8) connector having a mating surface (20) at which a terminal (18a,b) is provided connected to an end of the transmission line (14) for coupling with the signal terminal (48a,b) of the second conduit (44) provided on a mating surface (46) of a complementary female or male (44) connector of the second conduit (44) the terminals (18a,b;48a,b) being located on their respective connectors (8;44)) to establish a coupling when the mating surfaces (20;46) are engaged to provide the fluid coupling.

3. A conduit (2,22) as claimed in any preceding claim
**characterised in that** the distal end of the transmission line (14,36) is connectable to a sensor (28) for sensing a fluid parameter and for generating a signal dependent thereon and in that the proximal end of the transmission line (14,36) is connectable with a unit (50) for receiving the generated signal and providing an output dependent thereon.

4. A fluid transfer system comprising a fluid transfer device (40); a conduit (2,22) connectable in a flow path (44,2,22,44) between the transfer device (40) and internal a patient to provide for the through flow of a fluid; a sensor (28) for monitoring parameters of the fluid and for generating a signal dependent thereon; a processing unit (50) adapted to receive the generated signal and to provide an output signal dependent thereon; and a transmission line (14,36) incorporated with the conduit (2,22) having a distal end for connection to the fluid parameter sensor (28) and a proximal end for connection to the processing unit (50) to provide a transmission path for signals between the processing unit (50) and the sensor (28)
**characterised in that** the fluid transfer device (40) is provided with a port (44) for transferring fluid between internal and external the device having at an externally accessible surface (46) a terminal (48a,b) connected to the processing unit (50); and in that an interface (8) is provided in permanent connection with a proximal end of the conduit (2,22) for establishing concomitantly a coupling of the transmission line (14,36) with the terminal (48a,b) of the port (44) and a fluid coupling of the proximal end of the conduit (2,22) with the port(44).

5. A system as claimed in claim 4 **characterised in that** the interface (8) comprises a male or female connector having a mating surface (20) at which a terminal (18a,b) is provided connected to an end of the transmission line (14,36) for coupling with the terminal (48a,b) provided on the externally accessible complementary mating surface (46) of the port (44), the terminals (18a,b;48a,b) being located on their respective mating surfaces (20,46) to establish a coupling when the surfaces (20,46) are engaged to provide the fluid coupling.

6. A system as claimed in any of the claims 4 and 5
**characterised in that** the fluid transfer device (40) is adapted to receive the output from the processing unit (50) and to regulate fluid transfer in response thereto.

7. A system as claimed in any of the claims 4 to 6
**characterised in that** there is further provided a detector (56) in electrical communication with the terminal (48a,b) of the port (44) and adapted to detect the connection of the sensor (28) to the transmission line (14,36).
